# DEMANDE DE BREVET EUROPEEN

(11) **EP 4 475 097 A1**
(43) Date de publication de la demande: **11.12.2024**
(21) Numéro de dépôt: 24178803.3
(22) Date de dépôt: 29.05.2024
(51) Int. Cl.: G07C 9/00, G16H 40/20, G16H 80/00, G16H 40/67

(54) **CABINET PRIVATISABLE POUR TÉLÉCONSULTATION MÉDICALE ET PROCÉDÉ DE PRIVATISATION SÉCURISÉE DE LA CABINE**

(30) Priorité: 30.05.2023 FR 2305340
(71) Demandeur: Exo Sante, 30200 Bagnols-sur-Ceze (FR)
(72) Inventeur: TICHADOU, Christophe, 30200 VENEJAN (FR); DUCOS, Elodie, 69390 VERNAISON (FR)
(74) Mandataire: Fache, Sébastien

(57) **Abrégé**

L'invention porte principalement sur une cabine privatisable (1) pour effectuer une téléconsultation médicale, comprenant un bâti (2) et une porte d'accès (3), des moyens de condamnation (4) de la porte (3) actionnables entre un état verrouillé et un état déverrouillé, une borne de téléconsultation médicale (5) configurée pour être reliée à un réseau informatique distant, des moyens de détection d'un utilisateur (6) à l'intérieur de la cabine (1), des moyens de commande (8) reliés aux moyens de condamnation (4) de la porte (3) et aux moyens de détection de l'utilisateur (6), et qui sont configurés pour générer un niveau de corrélation entre des données issues d'une part des moyens de détection (6, 7) et d'autre part des moyens de condamnation (4) et, si le niveau de corrélation est inférieur à un seuil déterminé, pour générer un signal d'alerte et l'envoyer par le réseau distant.

## Description

### DOMAINE TECHNIQUE

L'invention s'inscrit dans le domaine des cabines privatisables installés dans des lieux recevant du public.

L'invention porte plus particulièrement sur l'accès sécurisé à la cabine et à son entretien entre deux accès.

### ART ANTERIEUR ET INCONVENIENTS DE L'ART ANTERIEUR

Devant la pénurie de professionnels de santé qui touchent la plupart des pays, les consultations à distance - ou téléconsultations - depuis le domicile du patient sont devenues courantes. Elles permettent à toutes personnes disposant d'un appareil informatique type tablette ou micro-ordinateur de consulter un médecin à distance.

Ces téléconsultations restent cependant limitées à des situations non urgentes, voire à des conseils génériques de santé.

Pour assurer des téléconsultations plus poussées et plus fiables, il existe des bornes de téléconsultations - ou bornes de télémédecine - disposant de moyens de communication avec le médecin mais également de moyens de mesure de paramètres biologiques du patient utilisateur, comme par exemple la tension artérielle ou la température corporelle.

Bien que fiables, ces bornes de téléconsultation nécessitent souvent la présence d'au moins un auxiliaire de santé pour des raisons de sécurité.

Pratiquement, les bornes de téléconsultation sont généralement installées dans des lieux qui permettent une intervention rapide d'un tel auxiliaire de santé en cas d'urgence, typiquement des pharmacies. L'espace disponible pour la pharmacie est donc réduit. Par ailleurs, une forte responsabilité incombe aux pharmaciens et auxiliaires de santé présents.

### OBJECTIF DE L'INVENTION

L'invention vise ainsi à proposer une cabine privatisable pour un téléconsultation médicale qui résout les inconvénients cités ci-dessus.

### EXPOSE DE L'INVENTION

A cet effet, l'invention concerne une cabine privatisable pour effectuer une téléconsultation médicale, comprenant un bâti et une porte d'accès, des moyens de condamnation de la porte actionnables entre un état verrouillé et un état déverrouillé, une borne de téléconsultation médicale configurée pour être reliée à un réseau informatique distant, des moyens de détection d'un utilisateur à l'intérieur de la cabine, des moyens de commande reliés aux moyens de condamnation de la porte et aux moyens de détection de l'utilisateur, et qui sont configurés pour générer un niveau de corrélation entre des données issues d'une part des moyens de détection et d'autre part des moyens de condamnation et, si le niveau de corrélation est inférieur à un seuil déterminé, pour générer un signal d'alerte et l'envoyer par le réseau distant.

La cabine de l'invention peut également comporter les caractéristiques optionnelles suivantes considérées isolément ou selon toutes les combinaisons techniques possibles :
- La cabine comprend des moyens de nettoyage de l'intérieur de la cabine pilotés par les moyens de commande.
- Les moyens de nettoyage comprennent des moyens d'irradiation ultraviolette reliés à des moyens d'alimentation en électricité pilotés par les moyens de commande.
- La cabine comprend des moyens de communication vocale configurés pour être reliés au réseau distant.
- Les moyens de détection comprennent au moins un capteur de présence d'un utilisateur à l'intérieur de ladite cabine.
- Le capteur de présence est monté sur un support réglable, lequel support est monté mobile sur une paroi du bâti de la cabine.
- La cabine comprend au moins deux capteurs de présence orientés selon deux directions différentes de manière à surveiller tout l'espace intérieur de la cabine.

L'invention vise également un procédé de privatisation sécurisée d'une cabine telle que décrite précédemment, lequel procédé comprend au moins les étapes successives de :
- Actionnement par un utilisateur des moyens de condamnation vers leur configuration déverrouillée, suivi de l'ouverture de la porte ;
- Fermeture de la porte et verrouillage des moyens de condamnation par les moyens de commande après un premier temps déterminé ;
- Actionnement des moyens de détection de l'utilisateur à l'intérieur de la cabine ;
   ∘ Si les moyens de détection détectent la présence de l'utilisateur dans la cabine, les moyens de commande assignent à la cabine un état occupé ;
   ∘ Si les moyens de détection détectent une absence de l'utilisateur dans la cabine, les moyens de commande concluent à une anomalie, assignent à la cabine un état anormal, puis génèrent et envoient une alerte par le réseau informatique distant.

Le procédé de l'invention peut également comporter les caractéristiques optionnelles suivantes considérées isolément ou selon toutes les combinaisons techniques possibles :
- La cabine étant dans son état occupé, le procédé comprend au moins les étapes supplémentaires successives de :
   - Ouverture de la porte ;
   - Fermeture de la porte et verrouillage des moyens de condamnation par les moyens de commande après un second temps déterminé ;
   - Actionnement des moyens de détection de l'utilisateur à l'intérieur de la cabine ;
      ∘ Si les moyens de détection confirment l'absence de l'utilisateur à l'intérieur de la cabine, les moyens de commande assignent à la cabine l'état vide ;
      ∘ Si les moyens de détection détectent la présence de l'utilisateur à l'intérieur de la cabine, les moyens de commande concluent à une anomalie, assignent à la cabine l'état anormal, puis génèrent et envoient une alerte par le réseau informatique distant.
- La cabine comprend des moyens d'irradiation ultraviolette reliés à des moyens d'alimentation en électricité pilotés par les moyens de commande, lequel procédé comprend, suite à l'étape de fermeture de la porte et verrouillage des moyens de condamnation et si les moyens de détection confirment l'absence de l'utilisateur à l'intérieur de la cabine, une étape de nettoyage par irradiation ultraviolette pendant un troisième temps déterminé.

### PRESENTATION DES FIGURES

D'autres caractéristiques et avantages de l'invention ressortiront clairement de la description qui en est donnée ci-dessous, à titre indicatif et nullement limitatif, en référence à la figure annexée :

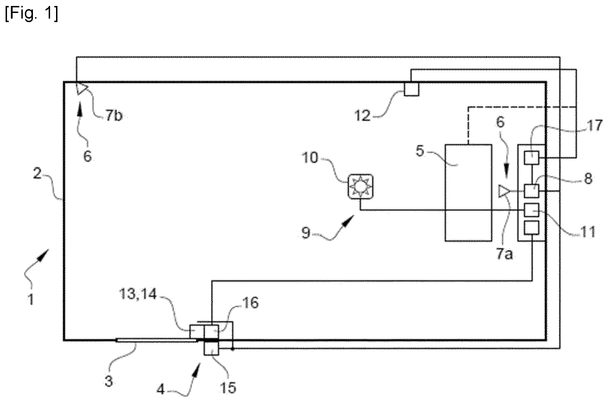

[Fig. 1] La figure 1 représente une vue schématique de la cabine privatisable de l'invention.

### DESCRIPTION DETAILLEE DE L'INVENTION

Il est tout d'abord précisé que la figure représente essentiellement un mode de réalisation de l'objet de l'invention mais qu'il peut exister d'autres modes de réalisation qui répondent à la définition de l'invention.

La présente invention concerne une cabine privatisable pour effectuer une téléconsultation médicale en toute sécurité.

En référence à la figure 1, la cabine 1 comprend un châssis 2, typiquement un système modulaire de construction, ainsi qu'une porte d'accès 3 et des moyens de condamnation automatique 4 de cette porte d'accès 3. Les moyens de condamnation 4 comprennent une gâche électrique 13 pilotée par un relais 14 alimenté en électricité, lequel relais est relié électriquement à une interface homme/machine 15, en particulier un digicode. La gâche 13 est actionnable entre un état déverrouillé dans lequel la porte 3 est actionnable entre un état ouvert et un état fermé, et un état verrouillé dans lequel la porte 3 est dans un état fermé et ne peut être actionnée vers son état ouvert. On entend par état ouvert de la porte 3 le fait que cette dernière soit dans un état permettant à un patient - ou utilisateur - de pénétrer dans la cabine 1, contrairement à l'état fermée de la porte 3 qui obstrue l'accès à l'intérieur de la cabine. La porte 3 comprend en outre des moyens d'ouverture disposés à l'intérieur de la cabine 1 - en particulier une barre antipanique - qui permet à l'utilisateur à l'intérieur de la cabine d'ouvrir la porte 3, même si les moyens de condamnation 4 sont verrouillés, par exemple en cas de coupure d'électricité.

Enfin, la porte 3 comprend des moyens de détection 16 de l'état ouvert ou fermé de la porte 3 par rapport au châssis 2 de la cabine 1.

La cabine privatisable 1 comprend également des moyens de commande 8 reliés via un routeur internet 17 à un réseau informatique distant principal. Ces moyens de commande informatiques 8 comprennent par exemple une carte électronique comportant un processeur et au moins un espace mémoire dans lequel au moins un logiciel de commande des différents éléments de la cabine 1 est installé. Le digicode 15 et le relais électrique 14 pilotant la gâche 13 est reliée à ces moyens de commande 8 de la cabine 1.

La cabine 1 comprend en outre une borne de téléconsultation médicale 5 qui est reliée via un second réseau informatique distant à une plate-forme de gestion de rendez-vous médicaux. Ce second réseau informatique est distinct du réseau informatique principal, de manière à conserver confidentielle, le cas échéant, l'identité des praticiens de santé. Dans certains mode de réalisation, une passerelle peut exister entre le réseau informatique principal et le second réseau informatique.

La cabine 1 comprend également des moyens de détection 6 d'un utilisateur à l'intérieur de la cabine 1.

De manière particulièrement avantageuse, les moyens de détection 1 comprennent au moins deux capteurs de détection 7a, 7b, par exemple infra-rouge ou sonores, reliés aux moyens de commande 8 de la cabine 1, et qui sont disposés de sorte à ne laisser aucun angle mort dans la zone de détection à l'intérieur de la cabine 1.

Pour encore améliorer la zone de couverture des capteurs 7a, 7b, ceux-ci sont montés sur des supports réglables et orientables sur les parois du châssis 2 de la cabine 1, y compris les parois de plafond et de plancher.

La cabine 1 comprend également des moyens de communication vocale 12 - par exemple un interphone installé dans la cabine 1 et comprenant au moins un haut-parleur et un microphone - reliés au routeur 17. Ces moyens de communications vocale présentent l'avantage de demeurer fonctionnels même en cas de panne électrique.

Enfin, la cabine 1 comprend des moyens de nettoyage 9 de l'intérieur de la cabine 1, idéalement des moyens d'irradiation ultraviolette 10 reliés à une prise 11 connectée aux moyens de commande 8. Ces moyens d'irradiation 9 sont prévus pour éliminer les germes, bactéries et virus à l'intérieur de la cabine 1 entre deux téléconsultations. Par ailleurs, ces moyens d'irradiation ultraviolette 10 sont positionnés de manière à maximiser la surface et le volume d'irradiation dans la cabine 1.

Les moyens de commande 8 de la cabine 1 sont configurés pour générer un niveau de corrélation entre les données renvoyées par les capteurs de présence 7a, 7b, les données renvoyées par les moyens de condamnation 4 et le cas échéant les données renvoyées par les moyens de nettoyage 9. Ces moyens de commande 8 sont en outre configurés pour générer un signal d'alerte et l'envoyer par le réseau distant si le niveau de corrélation généré est inférieur à un seuil déterminé.

En particulier, le niveau de corrélation sera inférieur au seuil déterminé - ce qui conduira à la génération d'une alerte - dans les cas suivants :
- Les moyens de détection 6 détectent la présence d'un utilisateur dans la cabine 1 et les moyens de commande 8 détectent une puissance non nulle au niveau de la prise connectée 11 des moyens d'irradiation ultraviolette 10 ;
- Les moyens de commande 8 détectent une puissance non nulle au niveau de la prise connectée 11 des moyens d'irradiation ultraviolette 10 et détectent que les moyens de condamnation 4 sont dans un état non verrouillé et/ou que la porte 3 est ouverte ;
- Les moyens de détection 6 détectent la présence d'un utilisateur dans la cabine et les moyens de commande 8 détectent que les moyens de condamnation 4 sont dans un état non verrouillé et/ou que la porte 3 est ouverte.

Dans les autres cas, le niveau de corrélation sera supérieur au seuil déterminé.

L'invention concerne également une interface de réservation à distance, qui permet aux utilisateurs de réserver un créneau de consultation avec un praticien de santé dans une cabine 1. Cette interface de réservation est reliée à la plate-forme de gestion de rendez-vous médicaux de chaque borne de téléconsultation 5 de chaque cabine 1 déployée. Avantageusement, cette interface est apte à envoyer également des rappels de rendez-vous, par exemple par messagerie électronique.

L'invention concerne également un système de gestion à distance de la cabine 1, et préférentiellement d'une pluralité de cabines privatisables 1, chacune comprenant une borne de téléconsultation médicale 5. Ce système de gestion est relié aux moyens de commande 8 via le routeur 17 de chaque cabine 1, à la plate-forme de gestion de rendez-vous médicaux de chaque borne de téléconsultation médicale 5 et à l'interface de réservation à distance.

Le système de gestion est en particulier configuré pour récupérer toutes les données générées par la cabine 1, c'est-à-dire les données issues des capteurs de détection 7a, 7b, des moyens de condamnation 4, de l'interphone 12, des moyens de nettoyage 9, ainsi que les alertes générées par les moyens de commande 8 de la cabine considérée 1. Le système de gestion récupère également les informations relatives aux rendez-vous médicaux, et est apte à relayer des alertes de sécurité utilisateur en cas de besoin.

L'invention concerne également un procédé de privatisation sécurisée de la cabine 1 pour réaliser une téléconsultation.

Au cours d'une première étape, l'utilisateur se connecte via un dispositif informatique - par exemple un micro-ordinateur ou une tablette - sur l'interface de réservation en ligne et réserve un créneau à la fois auprès d'un praticien de santé et pour l'occupation d'une cabine 1 selon l'invention pour une téléconsultation médicale. Alternativement, seul un créneau pour l'occupation de la cabine est réservable via la plateforme, et la réservation d'une consultation d'un praticien de santé est réalisée selon un autre processus, via une plateforme spécifique de réservation médicale. Une fois la réservation confirmée, l'utilisateur reçoit une confirmation du rendez-vous ainsi qu'un code à utiliser pour déverrouiller les moyens de condamnation de la cabine considérée 1. Par ailleurs, les informations relatives à cette réservation - c'est-à-dire au moins l'identifiant de la cabine réservée 1, le cas échéant le nom du praticien, les coordonnées de l'utilisateur et la plage horaire, le code pour déverrouiller les moyens de condamnation 4 de la cabine réservée 1 - sont envoyées au système de gestion de la ou des cabines de téléconsultation 1.

Au jour et à l'heure du rendez-vous, la cabine 1 étant dans un état vide et accessible, l'utilisateur au cours d'une seconde étape du procédé se présente devant la cabine 1 réservée et tape son code sur le digicode 15. Si le code est conforme, les moyens de commande 8 de la cabine 1 déverrouillent les moyens de condamnation 4 : le relais 14 est actionné et la gâche 13 est libérée. L'utilisateur ouvre la porte 3 et pénètre dans la cabine 1. Alternativement, l'ouverture de la porte 3 est automatique et pilotée par les moyens de commande 8 de la cabine 1. Si la gâche 13 est libérée et la porte 3 s'ouvre avant que le code ne soit tapé, ou si la porte 3 ne s'ouvre pas malgré l'entrée du code conforme, les moyens de commande 8 concluent à une anomalie de fonctionnement et génèrent une alerte de maintenance de la cabine 1 transmise aux moyens de gestion.

Au cours d'une troisième étape, après un premier temps déterminé, par exemple 15 secondes, les moyens de commande 8 pilotent la fermeture de la porte 3 et actionnent les moyens de condamnation 4 vers leur position de verrouillage. Ce verrouillage intervient donc sans action de l'utilisateur. Alternativement, si la fermeture de la porte 3 est manuelle, et que cette porte 3 n'a pas été fermée par l'utilisateur après l'écoulement du premier temps déterminé, les moyens de commande 8 concluent à une anomalie de fonctionnement et génèrent une alerte de maintenance transmise aux moyens de gestion.

Au cours d'une quatrième étape, les moyens de commande 8 actionnent les capteurs de détection 7a, 7b de l'utilisateur.

Si les capteurs 7a, 7b ne détectent aucun bruit et/ou mouvement au bout d'un second temps déterminé, alors les moyens de commande 8 concluent à une anomalie liée au comportement du patient, assignent à la cabine 1 un état anormal, puis génèrent et envoient une alerte liée au comportement du patient vers le système de gestion. Dans ce cas précis, le système de gestion peut établir une liaison via le routeur internet 17 de la cabine 1 avec les moyens de commande 8 pour émettre un signal audio qui sera diffusé par le haut-parleur de l'interphone 12. Sans réponse de la part du patient, des secours sont envoyés sur place.

Si les capteurs 7a, 7b détectent au moins un mouvement et/ou au moins un bruit dans la cabine 1, les moyens de commande 8 assignent à la cabine l'état occupé par un utilisateur, qui réalise alors sa téléconsultation.

Au cours d'une cinquième étape optionnelle, à la fin de la téléconsultation, la borne de téléconsultation 5 informe les moyens de commande 8, via la plateforme de gestion de rendez-vous médicaux, que le rendez-vous est terminé.

Les moyens de commande 8 pilotent l'ouverture de la porte 3.

Alternativement et de manière préférentielle, l'ouverture de la porte 3 est manuelle et réalisée par l'utilisateur. Dans tous les cas, si la porte 3 ne s'ouvre pas après un troisième temps déterminé, les moyens de commande 8 concluent à une anomalie de fonctionnement et génèrent une alerte de maintenance transmise aux moyens de gestion.

Une fois la porte 3 ouverte, les moyens de commande 8 pilotent, après un quatrième temps déterminé, la fermeture de la porte 3 et le verrouillage des moyens de condamnation 4.

Au cours d'une sixième étape, les moyens de commande actionnent les capteurs de détection 7a, 7b de l'utilisateur.

Si les capteurs 7a, 7b détectent au moins un bruit et/ou un mouvement dans la cabine 1, alors les moyens de commande 8 concluent à une anomalie liée au comportement du patient, assignent à la cabine 1 un état anormal, puis génèrent et envoient une alerte liée au comportement du patient vers le système de gestion. Dans ce cas précis, le système de gestion peut établir une liaison via le routeur internet 17 de la cabine 1 avec les moyens de commande 8 pour émettre un signal audio qui sera diffusé par le haut-parleur de l'interphone 12. Sans réponse de la part du patient, des secours sont envoyés sur place.

Si les capteurs 7a, 7b ne détectent pas de mouvement et/ou de bruit dans la cabine 1, les moyens de commande 8 assignent à la cabine 1 l'état vide et inaccessible par un utilisateur.

Enfin, au cours d'une dernière étape, les moyens de commande 8 pilotent l'actionnement des moyens d'irradiation ultraviolette 10 pendant un cinquième temps déterminé, et assignent à la cabine 1 l'état en nettoyage. A l'issue de ce cinquième temps, les moyens de commande 8 coupent l'alimentation des moyens d'irradiation 10 et assignent à la cabine 1 l'état vide et accessible à un utilisateur. Cette information est envoyée aux moyens de gestion.

Si durant la période de nettoyage, les moyens de commande 8 ne détectent pas de puissance au niveau de la prise connectée 11 des moyens d'irradiation 9, et/ou si les moyens de commande 8 détectent un déverrouillage des moyens de condamnation 4 et/ou une ouverture de la porte 3 et/ou une durée d'irradiation différente de la durée d'irradiation prévue, alors les moyens de commande 8 pilotent l'arrêt de l'alimentation des moyens d'irradiation 10 et concluent à une anomalie de fonctionnement de la cabine 1 et génèrent une alerte de maintenance transmise aux moyens de gestion.

Si durant la période de nettoyage les capteurs 7a, 7b détectent un utilisateur à l'intérieur de la cabine 1, alors les moyens de commande 8 pilotent l'arrêt de l'alimentation des moyens d'irradiation 10, concluent à une anomalie liée au comportement du patient et assignent à la cabine 1 un état anormal. Les moyens de commande génèrent et envoient une alerte liée au comportement du patient vers le système de gestion. Dans ce cas précis, le système de gestion peut établir une liaison via le routeur internet 17 de la cabine 1 avec les moyens de commande 8 pour émettre un signal audio qui sera diffusé sur le haut-parleur de l'interphone 12. Sans réponse de la part du patient, des secours sont envoyés sur place.

La cabine 1 de l'invention et le système de gestion d'une pluralité de ces cabines 1 permettent de faciliter les téléconsultations, en les rendant plus sécurisées sans nécessiter de personnels de santé et/ou de sécurité sur place durant les téléconsultations.

## Revendications

1. Cabine privatisable (1) pour effectuer une téléconsultation médicale, comprenant un bâti (2) et une porte d'accès (3), des moyens de condamnation (4) de la porte (3) actionnables entre un état verrouillé et un état déverrouillé, une borne de téléconsultation médicale (5) configurée pour être reliée à un réseau informatique distant, des moyens de détection d'un utilisateur (6) à l'intérieur de la cabine (1), des moyens de commande (8) reliés aux moyens de condamnation (4) de la porte (3) et aux moyens de détection de l'utilisateur (6), et qui sont configurés pour générer un niveau de corrélation entre des données issues d'une part des moyens de détection (6, 7) et d'autre part des moyens de condamnation (4) et, si le niveau de corrélation est inférieur à un seuil déterminé, pour générer un signal d'alerte et l'envoyer par le réseau distant.

2. Cabine (1) selon la revendication précédente, **caractérisée en ce que** qu'elle comprend des moyens de nettoyage (9) de l'intérieur de la cabine pilotés par les moyens de commande (8).

3. Cabine (1) selon la revendication précédente, **caractérisée en ce que** les moyens de nettoyage (9) comprennent des moyens d'irradiation ultraviolette (10) reliés à des moyens d'alimentation en électricité (11) pilotés par les moyens de commande (8).

4. Cabine (1) selon l'une quelconques des revendications 1 à 3, **caractérisée en ce qu'**elle comprend des moyens de communication vocale (12) configurés pour être reliés au réseau distant.

5. Cabine (1) selon l'une quelconque des revendications précédentes, **caractérisée en ce que** les moyens de détection (6) comprennent au moins un capteur de présence (7a, 7b) d'un utilisateur à l'intérieur de ladite cabine (1).

6. Cabine (1) selon la revendication précédente, **caractérisé en ce que** le capteur de présence (7a, 7b) est monté sur un support réglable, lequel support est monté mobile sur une paroi du bâti (2) de la cabine (1).

7. Cabine selon la revendication 5 ou 6, **caractérisée en ce qu'**elle comprend au moins deux capteurs de présence (7a, 7b) orientés selon deux directions différentes de manière à surveiller tout l'espace intérieur de la cabine (1).

8. Procédé de privatisation sécurisée d'une cabine (1) selon l'une quelconque des revendications 1 à 7, la cabine (1) étant vide et la porte d'accès (3) verrouillée, lequel procédé comprend au moins les étapes successives de :
• Actionnement par un utilisateur des moyens de condamnation (4) vers leur configuration déverrouillée, suivi de l'ouverture de la porte (3) ;
• Fermeture de la porte (3) et verrouillage des moyens de condamnation (4) par les moyens de commande après un premier temps déterminé ;
• Actionnement des moyens de détection de l'utilisateur (6) à l'intérieur de la cabine (1) ;
∘ Si les moyens de détection (6) détectent la présence de l'utilisateur dans la cabine (1), les moyens de commande (8) assignent à la cabine (1) un état occupé ;
∘ Si les moyens de détection (6) détectent une absence de l'utilisateur dans la cabine (1), les moyens de commande (8) concluent à une anomalie, assignent à la cabine (1) un état anormal, puis génèrent et envoient une alerte par le réseau informatique distant ;

9. Procédé selon la revendication précédente, **caractérisé en ce que**, la cabine (1) étant dans son état occupé, le procédé comprend les étapes supplémentaires successives de :
• Ouverture de la porte (3) ;
• Fermeture de la porte (3) et verrouillage des moyens de condamnation (4) par les moyens de commande (8) après un second temps déterminé ;
• Actionnement des moyens de détection de l'utilisateur (6) à l'intérieur de la cabine (1) ;
∘ Si les moyens de détection (6) confirment l'absence de l'utilisateur à l'intérieur de la cabine (1), les moyens de commande (8) assignent à la cabine (1) l'état vide ;
∘ Si les moyens de détection (6) détectent la présence de l'utilisateur à l'intérieur de la cabine (1), les moyens de commande (8) concluent à une anomalie, assignent à la cabine (1) l'état anormal, puis génèrent et envoient une alerte par le réseau informatique distant.

10. Procédé selon la revendication 8 ou 9, **caractérisé en ce que** la cabine (1) comprend des moyens d'irradiation ultraviolette (10) reliés à des moyens d'alimentation en électricité (11) pilotés par les moyens de commande (8), lequel procédé comprend, suite à l'étape de fermeture de la porte (3) et de verrouillage des moyens de condamnation (4), et si les moyens de détection (6) confirment l'absence de l'utilisateur à l'intérieur de la cabine (1), une étape de nettoyage par irradiation ultraviolette pendant un troisième temps déterminé.
